# EUROPÄISCHE PATENTANMELDUNG

(11) **EP 4 183 312 A1**
(43) Veröffentlichungstag der Anmeldung: **24.05.2023**
(21) Anmeldenummer: 22202775.7
(22) Anmeldetag: 20.10.2022
(51) Int. Cl.: A61B 1/00, A61B 1/05, G02B 23/24, A61B 1/06, A61B 1/07

(54) **SENSORVORRICHTUNG UND INSPEKTIONSKAMERA MIT EINER SOLCHEN SENSORVORRICHTUNG**

(30) Priorität: 19.11.2021 DE 102021213018
(71) Anmelder: Robert Bosch GmbH, 70442 Stuttgart (DE)
(72) Erfinder: Sgarz, Heiko, 71229 Leonberg (DE); Huembert, Frank, 70597 Stuttgart (DE); Brosi, Jan-Michael, 70771 Leinfelden-Echterdingen (DE)

(57) **Zusammenfassung**

Die Erfindung geht aus von einer Sensorvorrichtung, insbesondere einem Sensorkopf, für eine Inspektionskamera, insbesondere Endoskop, mit zumindest einer Beleuchtungseinheit (14a; 14b; 14c; 14d; 14e) zu einer Beleuchtung eines Untersuchungsobjekts, mit zumindest einer Kameraeinheit (20a; 20b; 20c; 20d; 20e) zu einer Erfassung des Untersuchungsobjekts und mit zumindest einer Gehäuseeinheit (22a; 22b; 22c; 22d; 22e), in welcher die Kameraeinheit (20a; 20b; 20c; 20d; 20e) und die Beleuchtungseinheit (14a; 14b; 14c; 14d; 14e) angeordnet sind.

Es wird vorgeschlagen, dass die Gehäuseeinheit (22a; 22b; 22c; 22d; 22e) entlang einer Längsachse (24a; 24b; 24c; 24d; 24e) der Gehäuseeinheit (22a; 22b; 22c; 22d; 22e) verjüngend ausgebildet ist.

## Beschreibung

### Stand der Technik

In der US 8,218,074 B2 ist bereits eine Sensorvorrichtung, insbesondere ein Sensorkopf, für eine Inspektionskamera, insbesondere für ein Endoskop, mit zumindest einer Beleuchtungseinheit zu einer Beleuchtung eines Untersuchungsobjekts, mit zumindest einer Kameraeinheit zu einer Erfassung des Untersuchungsobjekts und mit zumindest einer Gehäuseeinheit, in welcher die Kameraeinheit und die Beleuchtungseinheit angeordnet sind, vorgeschlagen worden.

### Offenbarung der Erfindung

Die Erfindung geht aus von einer Sensorvorrichtung, insbesondere einem Sensorkopf, für eine Inspektionskamera, insbesondere für ein Endoskop, mit zumindest einer Beleuchtungseinheit zu einer Beleuchtung eines Untersuchungsobjekts, mit zumindest einer Kameraeinheit zu einer Erfassung des Untersuchungsobjekts und mit zumindest einer Gehäuseeinheit, in welcher die Kameraeinheit und die Beleuchtungseinheit angeordnet sind.

Es wird vorgeschlagen, dass die Gehäuseeinheit entlang einer Längsachse der Gehäuseeinheit verjüngend ausgebildet ist. Die Sensorvorrichtung ist insbesondere dazu ausgebildet, für einen Menschen schwer oder nicht zugängliche Untersuchungsobjekte zerstörungsfrei zu erfassen. Das Untersuchungsobjekt ist beispielsweise eine Innenwand eines Rohrs, ein Hohlraum, eine Rückwand eines fest installierten Geräts, ein Innenleben, insbesondere ein Verdauungstrakt, eines Lebewesens oder dergleichen. Die Gehäuseeinheit weist bezüglich der Längsachse vorzugsweise einen vorderen Gehäuseabschnitt und einen hinteren Gehäuseabschnitt auf. Die Sensorvorrichtung ist insbesondere dazu vorgesehen, mit dem vorderen Gehäuseabschnitt voran in das Untersuchungsobjekt eingebracht, insbesondere eingeführt, zu werden. Die Gehäuseeinheit weist insbesondere zumindest eine Kabeldurchführung auf, die an dem hinteren Gehäuseabschnitt angeordnet ist. Die Kabeldurchführung ist insbesondere zu einer Aufnahme einer Kabeleinheit der Inspektionskamera vorgesehen. Die Kabeleinheit verbindet insbesondere die Kameraeinheit und/oder die Beleuchtungseinheit mit einer Energiequelle der Inspektionskamera. Die Kabeldurchführung ist vorzugsweise dazu vorgesehen, eine Zentralachse der Kabeleinheit an der Kabeldurchführung parallel zu der Längsachse der Gehäuseeinheit auszurichten. Insbesondere ist die Kabeldurchführung dazu vorgesehen, die Kabeleinheit und die Gehäuseeinheit bezüglich der Längsachse konzentrisch auszurichten.

Die Gehäuseeinheit weist an einer Außenseite zumindest eine Schrägfläche auf, welche eine Verjüngung der Gehäuseeinheit bezogen auf die Längsachse ausbildet. Die Schrägfläche und/oder eine Tangentialebene der Schrägfläche schließt mit der Längsachse insbesondere einen spitzen Winkel ein. Die Schrägfläche kann in dem vorderen Gehäuseabschnitt, in dem hinteren Gehäuseabschnitt und/oder zwischen dem vorderen und dem hinteren Gehäuseabschnitt angeordnet sein. Die Schrägfläche kann eben oder gekrümmt, insbesondere rotationssymmetrisch bezüglich der Längsachse, ausgebildet sein. Beispielsweise ist ein Teil der Gehäuseeinheit, insbesondere der vordere Gehäuseabschnitt und/oder der hintere Gehäuseabschnitt, oder die gesamte Gehäuseeinheit kegelförmig, kegelstumpfförmig, pyramidenförmig, pyramidenstumpfförmig, keilförmig, rotationshyperboloidförmig, rotationsparaboloidförmig, kugelförmig, halbkugelförmig oder dergleichen ausgebildet. Die Gehäuseeinheit kann sich kontinuierlich oder in mehreren Stufen verjüngen. Bilden sowohl der vordere Gehäuseabschnitt als auch der hintere Gehäuseabschnitt eine Verjüngung aus, können diese Verjüngungen in die gleiche Richtung oder in entgegengesetzte Richtungen entlang der Längsachse die Gehäuseeinheit verjüngen. Die Gehäuseeinheit weist vorzugsweise parallel zu der Längsachse, insbesondere entlang der Längsachse, eine maximale Längserstreckung auf. Vorzugsweise weist die Gehäuseeinheit senkrecht zu der Längsachse eine maximale Quererstreckung und eine von der maximalen Quererstreckung unterschiedliche minimale Quererstreckung auf.

Insbesondere verbindet die Schrägfläche die maximale Quererstreckung mit der minimalen Quererstreckung. Die maximale Quererstreckung kann in dem vorderen Gehäuseabschnitt, in dem hinteren Gehäuseabschnitt und/oder zwischen dem vorderen und dem hinteren Gehäuseabschnitt angeordnet sein. Die minimale Quererstreckung ist vorzugsweise in dem vorderen Gehäuseabschnitt oder in dem hinteren Gehäuseabschnitt angeordnet, insbesondere an einem Ende der Gehäuseeinheit entlang der Längsachse. Die maximale Längserstreckung kann größer oder kleiner als die maximale Quererstreckung und/oder die minimale Quererstreckung sein.

Die Schrägfläche erstreckt sich parallel zu der Längsachse vorzugsweise über einen wesentlichen Teil der maximalen Längserstreckung der Gehäuseeinheit. Unter einem "wesentlichen Teil" einer Bezugsstrecke, soll insbesondere zumindest 15 %, bevorzugt mehr als 25 %, besonders bevorzugt mehr als 33 %, optional mehr als 50 %, einer Gesamtlänge der Bezugsstrecke verstanden werden. Eine minimale Quererstreckung ist vorzugsweise um zumindest 1 %, vorzugsweise um mehr als 3 %, besonderes bevorzugt um mehr als 5 %, der maximalen Quererstreckung kleiner als die maximale Quererstreckung. In zumindest einer Ausgestaltung ist die minimale Quererstreckung insbesondere quasi-punktförmig und bildet beispielsweise einen Scheitelpunkt einer Wölbung oder einer Spitze. In einer alternativen Ausgestaltung ist die minimale Quererstreckung eine maximale Erstreckung einer Frontfläche der Gehäuseeinheit, insbesondere des vorderen Gehäuseabschnitts, welche insbesondere senkrecht zu der Längsachse verläuft.

Die Kameraeinheit und die Beleuchtungseinheit sind insbesondere im Inneren der Gehäuseeinheit angeordnet. Die Gehäuseeinheit umfasst insbesondere ein zusammenhängendes Außengehäuse, welches die Verjüngung ausbildet und in welchem die Kameraeinheit und die Beleuchtungseinheit gemeinsam angeordnet sind. Optional umfasst die Gehäuseeinheit zumindest ein Strukturelement, welches einen Innenraum des Außengehäuses in mehrere Teilräume unterteilt. Das Außengehäuse kann aus einem Stück gebildet sein oder aus mehreren Montageteilgehäusen, insbesondere Montagehalbschalen, zusammengesetzt ausgebildet sein. Die Gehäuseeinheit umfasst insbesondere zumindest eine Beleuchtungsöffnung, durch welche hindurch die Beleuchtungseinheit das Untersuchungsobjekt beleuchten kann. Alternativ oder zusätzlich ist die Gehäuseeinheit zumindest teilweise aus einem lichtdurchlässigen Material ausgebildet, insbesondere aus einem transparenten oder transluzenten Material. Die Gehäuseeinheit umfasst vorzugsweise zumindest eine Kameraöffnung zu einem Durchlassen von Licht durch die Gehäuseeinheit zu der Kameraeinheit. Die Kameraeinheit umfasst insbesondere zumindest eine Kameraelement, optional mehrere Kameraelemente. Das zumindest ein Kameraelement ist bevorzugt als Photodetektor ausgebildet, insbesondere als Active Pixel Sensor (CMOS-Sensor) oder als Charged-Coupled-Device-Sensor (CCD-Sensor). Die Beleuchtungseinheit umfasst vorzugsweise zumindest ein Beleuchtungselement und besonders bevorzugt zumindest ein weiteres Beleuchtungselement. Vorzugsweise umfasst die Beleuchtungseinheit zumindest eine anorganische oder organische Leuchtdiode (LED, OLED) als Beleuchtungselement und/oder als weiteres Beleuchtungselement. In einer vorteilhaft kompakten Ausgestaltung weisen die Beleuchtungseinheit und die Kameraeinheit eine gemeinsame Leiterplatte auf, an welcher das zumindest eine Kameraelement und das zumindest eine Beleuchtungselement angeordnet sind. Alternativ sind die Beleuchtungseinheit und die Kameraeinheit separat voneinander ausgebildet und insbesondere beabstandet voneinander innerhalb der Gehäuseeinheit, insbesondere auf verschiedenen Leiterplatten, angeordnet.

Durch die erfindungsgemäße Ausgestaltung der Sensorvorrichtung kann die Sensorvorrichtung vorteilhaft leicht innerhalb des Untersuchungsobjekts bewegt werden. Insbesondere kann eine Gleitbewegung der Sensorvorrichtung innerhalb des Untersuchungsobjekts vorteilhaft unterstützt werden. Insbesondere kann ein Risiko eines Verhakens und/oder Steckenbleibens der Sensorvorrichtung innerhalb des Untersuchungsobjekts vorteilhaft gering gehalten werden. Insbesondere kann ein Risiko einer Beschädigung der Sensorvorrichtung und/oder des Untersuchungsobjekts vorteilhaft gering gehalten werden.

Weiter wird vorgeschlagen, dass die Gehäuseeinheit bezüglich der Längsachse einen, insbesondere den bereits genannten, vorderen Gehäuseabschnitt, einen, insbesondere den bereits genannten, hinteren Gehäuseabschnitt und eine, insbesondere die bereits genannte, an dem hinteren Gehäuseabschnitt angeordnete Kabeldurchführung zu einem Anschluss der Kameraeinheit und/oder der Beleuchtungseinheit an eine, insbesondere die bereits genannte, Energiequelle der Inspektionskamera umfasst, wobei der vordere Gehäuseabschnitt entlang der Längsachse in einer von dem hinteren Gehäuseabschnitt abgewandten Richtung verjüngend ausgebildet ist. Insbesondere schließt der vordere Gehäuseabschnitt entlang der Längsachse mit der minimalen Quererstreckung ab. Der hintere Gehäuseabschnitt kann in zur Längsachse senkrechten Ebenen entlang der Längsachse eine konstante Quererstreckung, insbesondere die maximale Quererstreckung der Gehäuseeinheit, aufweisen oder eine variable Quererstreckung aufweisen. Durch die erfindungsgemäße Ausgestaltung kann ein Risiko eines Verhakens bei einem Einbringen der Sensorvorrichtung in das Untersuchungsobjekt vorteilhaft gering gehalten werden.

Ferner wird vorgeschlagen, dass die Gehäuseeinheit bezüglich einer zur Längsachse parallelen Mittenebene durch einen geometrischen Schwerpunkt der Gehäuseeinheit asymmetrisch ausgebildet ist. Insbesondere umfasst die Gehäuseeinheit zumindest eine parallel zu der Längsachse verlaufende Gehäusewand. Insbesondere liegen sich die Schrägfläche und die zur Längsachse parallele Gehäusewand bezüglich der Mittenebene gegenüber. Alternativ umfasst die Gehäuseeinheit zumindest eine weitere Schrägfläche als Außenwand, welche der Schrägfläche bezüglich der Mittenebene gegenüberliegt. Die weitere Schrägfläche oder eine Tangentialebene der weiteren Schrägfläche schließt beispielsweise mit der Längsachse einen weiteren spitzen Winkel ein, der unterschiedlich ist von dem spitzen Winkel zwischen der Schrägfläche oder der Tangentialebene der Schrägfläche und der Längsachse. Alternativ ist die Schrägfläche gekrümmt und die weitere Schrägfläche eben ausgebildet oder die Schrägfläche ist eben und die weitere Schrägfläche gekrümmt ausgebildet. Durch die erfindungsgemäße Ausgestaltung kann ein Umlenken der Sensorvorrichtung um eine Ecke oder Biegung innerhalb des Untersuchungsobjekts zumindest in eine Richtung vorteilhaft unterstützt werden. Darüber hinaus kann gleichzeitig ein vorteilhaft großer Innenraum innerhalb der Gehäuseeinheit beibehalten werden.

Weiterhin wird vorgeschlagen, dass ein eine, insbesondere die bereits genannte, Kabeldurchführung der Gehäuseeinheit begrenzender Rand der Gehäuseeinheit in einer zur Längsachse senkrechten Ebene eine, insbesondere die bereits genannte, maximale Quererstreckung der Gehäuseeinheit senkrecht zur Längsachse aufweist. Insbesondere ist die maximale Quererstreckung der Gehäuseeinheit unwesentlich größer als eine maximale Öffnungsweite der Kabeldurchführung. Unter "unwesentlich größer" soll insbesondere um weniger als ein Dreifaches einer maximalen Materialstärke der Gehäuseeinheit, bevorzugt um weniger als ein Doppeltes einer maximalen Materialstärke der Gehäuseeinheit, insbesondere um höchsten das Einfache einer maximalen Materialstärke der Gehäuseeinheit, größer verstanden werden. Vorzugsweise ist die maximale Quererstreckung der Gehäuseeinheit zumindest im Wesentlichen gleich groß wie eine maximale Kabelquererstreckung der Kabeleinheit. Unter "im Wesentlichen gleich" soll insbesondere gleich mit einer Abweichung von weniger als 10 %, bevorzugt von weniger als 5 %, insbesondere von weniger als 3 % verstanden werden. Insbesondere ist die Kabeldurchführung dazu vorgesehen, die Kabeleinheit und die Gehäuseeinheit zumindest im Wesentlichen bündig aneinander anzuordnen, insbesondere mit einem Versatz von weniger als 1 mm, bevorzugt weniger als 0,5 mm, senkrecht zur Längsachse aneinander anzuordnen. Durch die erfindungsgemäße Ausgestaltung kann vorteilhaft ein Absatz zwischen der Kabeleinheit und der Sensorvorrichtung vermieden werden. Insbesondere kann ein Risiko eines Verhakens bei einem Ausbringen der Sensorvorrichtung aus dem Untersuchungsobjekt vorteilhaft klein gehalten werden.

Ferner wird vorgeschlagen, dass die Gehäuseeinheit bezüglich der Längsachse einen, insbesondere den bereits genannten, vorderen Gehäuseabschnitt, einen, insbesondere den bereits genannten, hinteren Gehäuseabschnitt und eine, insbesondere die bereits genannte, an dem hinteren Gehäuseabschnitt angeordnete Kabeldurchführung zu einem Anschluss der Kameraeinheit und/oder der Beleuchtungseinheit an eine, insbesondere die bereits genannte, Energiequelle der Inspektionskamera umfasst, wobei der hintere Gehäuseabschnitt entlang der Längsachse in einer von dem vorderen Gehäuseabschnitt abgewandten Richtung verjüngend ausgebildet ist. Insbesondere weist der hintere Gehäuseabschnitt in einer zur Längsachse senkrechten Ebene, in welcher die Kabeldurchführung angeordnet ist, eine minimale hintere Quererstreckung auf. Die minimale hintere Quererstreckung kann identisch mit, gleich wie oder unterschiedlich von der minimalen Quererstreckung der gesamten Gehäuseeinheit ausgebildet sein. Die minimale hintere Quererstreckung ist insbesondere größer als die maximale Öffnungsweite der Kabeldurchführung. Die minimale hintere Quererstreckung ist vorzugsweise kleiner als die maximale Quererstreckung der Gehäuseeinheit. Insbesondere verbreitert sich die Gehäuseeinheit ausgehend von der Kabeldurchführung entlang der Längsachse bis zur maximalen Quererstreckung, insbesondere kontinuierlich. Der vordere Gehäuseabschnitt kann in zur Längsachse senkrechten Ebenen entlang der Längsachse eine konstante Quererstreckung, insbesondere die maximale Quererstreckung der Gehäuseeinheit, aufweisen oder eine variable Quererstreckung aufweisen. Durch die erfindungsgemäße Ausgestaltung kann vorteilhaft ein Absatz zwischen der Kabeleinheit und der Sensorvorrichtung vermieden werden. Insbesondere kann ein Risiko eines Verhakens bei einem Ausbringen der Sensorvorrichtung aus dem Untersuchungsobjekt vorteilhaft klein gehalten werden.

Des Weiteren wird vorgeschlagen, dass die Sensorvorrichtung zumindest ein die Gehäuseeinheit verschließendes Schutzfenster zu einem Schutz der Kameraeinheit und zumindest ein weiteres Schutzfenster zu einem Schutz der Beleuchtungseinheit umfasst, wobei das Schutzfenster und das weitere Schutzfenster separat voneinander ausgebildet sind. Das Schutzfenster ist insbesondere in der Kameraöffnung der Gehäuseeinheit angeordnet. Das Schutzfenster ist vorzugsweise aus einem transparenten Material gefertigt. Das weitere Schutzfenster ist insbesondere in der Beleuchtungsöffnung angeordnet. Das weitere Schutzfenster kann aus einem transparenten oder transluzenten Material gefertigt sein. Die Kameraöffnung und die Beleuchtungsöffnung der Gehäuseeinheit können einstückig ausgebildet sein, insbesondere eine einzelne Öffnung bilden, oder beabstandet voneinander angeordnet sein. Das Schutzfenster umfasst vorzugsweise eine Kamerafläche, welche der Kameraeinheit zugewandt ist, eine Objektfläche, welche dazu vorgesehen ist, dem Untersuchungsobjekt zugewandt zu werden, und zumindest eine Seitenwand, welche die Kamerafläche und die Objektfläche miteinander verbindet. Das weitere Schutzfenster umfasst vorzugsweise eine Beleuchtungsfläche, welche der Beleuchtungseinheit zugewandt ist, eine weitere Objektfläche, welche dazu vorgesehen ist, dem Untersuchungsobjekt zugewandt zu werden, und zumindest eine weitere Seitenwand, welche die Beleuchtungsfläche und die weitere Objektfläche miteinander verbindet. Die Schutzfenster können optional als optische Linsen ausgebildet sein. Vorzugsweise umfasst die Sensorvorrichtung zumindest ein Blockierelement, welche an der Seitenwand des Schutzfensters und/oder an der Seitenwand des weiteren Schutzfensters, insbesondere zwischen den Schutzfenstern angeordnet ist. Das Blockierelement ist insbesondere aus einem lichtundurchlässigen Material gefertigt. Das Blockierelement ist insbesondere dazu vorgesehen, gehäuseinternes Licht von der Beleuchtungseinheit zu blockieren, insbesondere durch Absorption und/oder Reflexion. Insbesondere ist das Blockierelement dazu vorgesehen, ein Einkoppeln von gehäuseinternem Licht von der Beleuchtungseinheit in die Kameraeinheit zu blockieren. Unter "gehäuseinternen" Licht soll insbesondere Licht verstanden werden, das innerhalb der Gehäuseeinheit erzeugt wird und ohne die Gehäuseeinheit zu verlassen von der Kameraeinheit, insbesondere als Streulicht, detektiert werden kann, insbesondere im Gegensatz zu Licht, das von dem Untersuchungsobjekt emittiert oder reflektiert wird. Das Blockierelement ist vorzugsweise als Strukturelement, insbesondere Abstandshalter, der Gehäuseeinheit ausgebildet. Alternativ ist das Blockierelement als auf der Seitenwand und/oder der weiteren Seitenwand aufgebrachte Folie oder Beschichtung ausgebildet. Durch die erfindungsgemäße Ausgestaltung kann eine Lichtmenge der Beleuchtungseinheit, welche nicht mit den Untersuchungsobjekt interagiert hat und auf die Kameraeinheit trifft, vorteilhaft gering gehalten werden. Insbesondere kann eine Leitung des Lichts von dem weiteren Schutzfenster zu dem Schutzfenster vorteilhaft gering gehalten werden.

Darüber hinaus wird vorgeschlagen, dass das Schutzfenster und das weitere Schutzfenster in einer Richtung parallel zu der Längsachse versetzt zueinander angeordnet sind. Das Schutzfenster weist insbesondere eine maximale Fensterdicke zwischen der Kamerafläche und der Objektfläche auf. Das weitere Schutzfenster weist insbesondere eine weitere maximale Fensterdicke zwischen der Beleuchtungsfläche und der weiteren Objektfläche auf. Vorzugsweise sind das Schutzfenster und das weitere Schutzfenster parallel zu der Längsachse um mehr als die, insbesondere um mehr als das Doppelte der, maximalen Fensterdicke und/oder um mehr als die, insbesondere um mehr als das Doppelte der, weiteren maximalen Fensterdicke versetzt angeordnet. Vorzugsweise ist dasjenige der Schutzfenster, welches näher an, insbesondere auf, der Längsachse angeordnet ist, in Richtung des vorderen Gehäuseabschnitts versetzt, während dasjenige der Schutzfenster, welches weiter entfernt von der Längsachse angeordnet ist, in Richtung des hinteren Gehäuseabschnitts versetzt ist. Durch die erfindungsgemäße Ausgestaltung kann der vordere Gehäuseabschnitt vorteilhaft stark verjüngt werden.

Weiter wird vorgeschlagen, dass eine Blickrichtung der Kameraeinheit unter einem, insbesondere einstellbaren, spitzen Winkel zu der Längsachse ausgerichtet ist, um das Untersuchungsobjekt durch eine, insbesondere die bereits genannte, die Gehäuseeinheit verjüngende Schrägfläche der Gehäuseeinheit hindurch zu erfassen. Die Blickrichtung ist insbesondere eine Zentralachse eines maximalen Sichtwinkels der Kameraeinheit, insbesondere des Kameraelements. Bei einer Ausgestaltung der Kameraeinheit mit mehreren Kameraelementen, umfasst vorzugsweise jedes Kameraelement eine eigene Blickrichtung, die parallel zueinander ausgerichtet, in einer gemeinsamen Blickebene angeordnet oder windschief zueinander angeordnet sein können. Die Blickrichtung des zumindest einen Kameraelements ist vorzugsweise zumindest im Wesentlichen senkrecht zu der Kamerafläche des Sichtfensters ausgerichtet, insbesondere ausrichtbar. Insbesondere ist die Kameraöffnung teilweise oder vollständig in der Schrägfläche angeordnet. Vorzugsweise ist die Blickrichtung zumindest im Wesentlichen senkrecht zu der Schrägfläche ausgerichtet, insbesondere ausrichtbar. In einer vorteilhaft einfachen Ausgestaltung ist der spitze Winkel, welcher die Blickrichtung mit der Längsachse einschließt, fix. Alternativ umfasst die Kameraeinheit zumindest ein Antriebselement, um einen Winkel der Blickrichtung relativ zu der Längsachse zu verändern, insbesondere zu schwenken. Das Antriebselement kann dazu vorgesehen sein, das Kameraelement relativ zu der Gehäuseeinheit zu bewegen, insbesondere zu schwenken, oder ein reflektierendes Optikelement zu bewegen, insbesondere zu schwenken. Das reflektierende Optikelement ist vorzugsweise ein Spiegel, insbesondere ein Mikrospiegelaktor. Bei einer Ausgestaltung der Kameraeinheit mit dem Antriebselement zum Ändern der Blickrichtung, umfasst eine mit dem Antriebselement einstellbare Winkelspanne für den Winkel zwischen der Blickrichtung und der Längsachse vorzugsweise auch eine zu der Längsachse parallele Ausrichtung der Blickrichtung und/oder optional eine zu der Längsachse senkrechte Ausrichtung der Blickrichtung. Alternativ ist die Blickrichtung der Kameraeinheit zumindest im Wesentlichen parallel oder zumindest im Wesentlichen senkrecht zu der Längsachse, insbesondere fix, ausgerichtet. Durch die erfindungsgemäße Ausgestaltung kann das Untersuchungsobjekt vorteilhaft genau untersucht werden.

Ferner wird vorgeschlagen, dass die Sensorvorrichtung eine in der Gehäuseeinheit angeordnete Montageplatte, insbesondere die bereits genannte Leiterplatte, umfasst, an welcher zumindest die Kameraeinheit montiert ist, wobei die Beleuchtungseinheit zumindest ein Beleuchtungselement, insbesondere das bereits genannte Beleuchtungselement, das bereits genannte weitere Beleuchtungselement oder ein zusätzliches Beleuchtungselement, umfasst, welches auf einer von der Kameraeinheit abgewandten Seite der Montageplatte angeordnet ist. Im Folgenden wird dieses Beleuchtungselement als rückwärtiges Beleuchtungselement bezeichnet. Die Kameraeinheit, insbesondere das zumindest eine Kameraelement, ist vorzugsweise dem vorderen Gehäuseabschnitt zugewandt angeordnet. Das rückwärtige Beleuchtungselement ist vorzugsweise dem hinteren Gehäuseabschnitt zugewandt angeordnet. Das rückwärtige Beleuchtungselement ist insbesondere als LED oder OLED ausgebildet. Vorzugsweise weist das rückwärtige Beleuchtungselement eine Hauptabstrahlrichtung auf, welche zumindest im Wesentlichen parallel zu einer Oberfläche der Montageplatte verläuft, auf der das rückwärtige Beleuchtungselement montiert ist. Die Hauptabstrahlrichtung des rückwärtigen Beleuchtungselements verläuft vorzugsweise quer, insbesondere senkrecht, zu der Längsachse. Insbesondere umfasst die Gehäuseeinheit eine rückwärtige Beleuchtungsöffnung, insbesondere die Beleuchtungsöffnung oder eine zusätzliche Beleuchtungsöffnung, durch welche Licht von dem rückwärtigen Beleuchtungselement aus der Gehäuseeinheit hinaus gestrahlt werden kann. Das rückwärtige Beleuchtungselement ist insbesondere zu einer indirekten Beleuchtung des Untersuchungsobjekts vorgesehen. Durch die erfindungsgemäße Ausgestaltung können direkte Reflexionen am Untersuchungsobjekt, welche zu einer Überbelichtung der Kameraeinheit führen können, vorteilhaft vermieden werden.

Weiterhin wird vorgeschlagen, dass die Gehäuseeinheit zumindest einen Lichtleiter ausbildet, welcher von der Beleuchtungseinheit parallel oder quer zur Längsachse wegführt. Die Gehäuseeinheit umfasst den Lichtleiter insbesondere anstelle des weiteren Schutzfensters. Der Lichtleiter ist beispielsweise als Lichtwellenleiter oder als planare Lichtwellenleiterstruktur (PLWL) ausgebildet. Der Lichtleiter ist insbesondere in die Beleuchtungsöffnung eingelassen. Durch die erfindungsgemäße Ausgestaltung kann vorteilhaft auf das weitere Sichtfenster verzichtet werden. Insbesondere kann die Beleuchtungsöffnung vorteilhaft klein gehalten werden. Insbesondere kann ein Risiko eines Eindringens von Fremdobjekten und/oder Schmutz in die Beleuchtungsöffnung vorteilhaft klein gehalten werden.

Des Weiteren wird vorgeschlagen, dass die Sensorvorrichtung zumindest einen Ausrichtungssensor zu einer Bestimmung einer räumlichen Ausrichtung der Kameraeinheit aufweist. Der Ausrichtungssensor ist vorzugsweise innerhalb der Gehäuseeinheit angeordnet. Alternativ ist der Ausrichtungssensor in die Kabeleinheit der Inspektionskamera integriert. Der Ausrichtungssensor ist vorzugsweise als inertiale Messeinheit (IMU) ausgebildet, welche vorzugsweise Drehratensensoren und/oder Beschleunigungssensoren umfasst. Der Ausrichtungssensor ist vorzugsweise dazu vorgesehen, eine Ausrichtung der Gehäuseeinheit, insbesondere der Kameraeinheit, relativ zur Schwerkraft zu erfassen. Der Ausrichtungssensor ist insbesondere dazu vorgesehen, einem von der Kameraeinheit erfassten Bild des Untersuchungsobjekts eine Ausrichtungsinformation zuzuordnen, welches eine Ausrichtung des Untersuchungsobjekts relativ zur Schwerkraft angibt. Eine Verarbeitung des von der Kameraeinheit erfassten Bildes und der von dem Ausrichtungssensor erfassten Ausrichtungsinformation erfolgt vorzugsweise von einer externen Recheneinheit, insbesondere einer Recheneinheit der Inspektionskamera, welche außerhalb der Gehäuseeinheit angeordnet ist. Alternativ umfasst die Sensorvorrichtung eine Recheneinheit, welcher in der Gehäuseeinheit angeordnet ist und das Bild und die Ausrichtungsinformation miteinander verknüpft. Die Ausrichtungsinformation kann beispielsweise durch eine Markierung, beispielsweise in Form eines Pfeils, eines Koordinatensystems oder dergleichen, innerhalb des Bildes zusammen mit dem Bild gespeichert und/ insbesondere ausgegeben werden oder, insbesondere von der Recheneinheit, dazu genutzt werden das Bild in eine Standardausrichtung zu drehen, insbesondere an eine Ausrichtung eines Displays zur Darstellung des Bildes automatisch anzupassen. Durch die erfindungsgemäße Ausgestaltung kann ein von der Kameraeinheit erfasstes Bild des Untersuchungsobjekts vorteilhaft einfach interpretiert werden. Insbesondere können Stellen des Untersuchungsobjekts von Interesse vorteilhaft einfach lokalisiert werden. Insbesondere kann die Sensorvorrichtung vorteilhaft intuitiv in dem Untersuchungsobjekt navigiert werden.

Darüber hinaus wird eine Inspektionskamera, insbesondere ein Endoskop, mit zumindest einer erfindungsgemäßen Sensorvorrichtung und mit zumindest einer von der Gehäuseeinheit beabstandet angeordneten Energiequelle zu einer Energieversorgung der Kameraeinheit und/oder der Beleuchtungseinheit mit elektrischer Energie vorgeschlagen. Die Inspektionskamera umfasst insbesondere eine Bedieneinheit, die bereits genannte Kabeleinheit und die Sensorvorrichtung. Insbesondere ist die Sensorvorrichtung, insbesondere nur, über die Kabeleinheit mit der Bedieneinheit verbunden. Die Bedieneinheit umfasst insbesondere ein Bediengehäuse, in welchem die Energiequelle angeordnet ist. Das Bediengehäuse ist vorzugsweise handhaltbar, insbesondere mit einer Hand und insbesondere ohne Hilfsmittel. Die Bedieneinheit umfasst bevorzugt eine Ausgabeeinheit. Die Ausgabeeinheit umfasst vorzugsweise ein Display zu einer Darstellung des mit der Kameraeinheit erfassten Bildes und/oder der mit dem Ausrichtungssensor erfassten Ausrichtungsinformation. Die Ausgabeeinheit ist insbesondere in das Bediengehäuse eingelassen. Vorzugsweise umfasst die Bedieneinheit die bereits genannte Recheneinheit. Unter einer "Recheneinheit" soll insbesondere eine Einheit mit einem Informationseingang, einer Informationsverarbeitung und einer Informationsausgabe verstanden werden. Vorteilhaft weist die Recheneinheit zumindest einen Prozessor, einen Speicher, Ein- und Ausgabemittel, weitere elektrische Bauteile, ein Betriebsprogramm, Regelroutinen, Steuerroutinen und/oder Berechnungsroutinen auf. Vorzugsweise sind die Bauteile der Recheneinheit auf einer gemeinsamen Platine angeordnet und/oder vorteilhaft in einem gemeinsamen Gehäuse angeordnet. Die Kabeleinheit umfasst insbesondere zumindest eine Stromleitung, welche die Kameraeinheit und/oder die Beleuchtungseinheit mit der Energiequelle verbindet. Die Energiequelle ist vorzugsweise als Akkumulator oder Batterie ausgebildet. Alternativ ist die Energiequelle als Stromanschluss ausgebildet, insbesondere zu einem Anschluss an ein externes Stromnetz. Die Bedieneinheit umfasst vorzugsweise zumindest ein Bedienelement, insbesondere zumindest zu einem Ein- und/oder Ausschalten der Sensorvorrichtung. Vorzugsweise umfasst die Kabeleinheit zumindest eine Datenleitung, welche die Kameraeinheit und/oder den Ausrichtungssensor mit der Recheneinheit verbindet. Die Kabeleinheit umfasst insbesondere eine Ummantelung, in welcher die Stromleitung und die Datenleitung der Kabeleinheit gemeinsam angeordnet sind. Die Ummantelung ist vorzugsweise als Schlauch, insbesondere als Wickelschlauch oder Wellschlauch, aus Metall oder Kunststoff ausgebildet. Vorzugsweise weist die Kabeleinheit, insbesondere in einem abgewickelten Zustand, eine größere, insbesondere mehr als fünfmal größere, vorzugsweise mehr als zehnmal, besonders bevorzugt mehr als zwanzigmal, größere maximale Längserstreckung als die Sensorvorrichtung auf. Durch die erfindungsgemäße Ausgestaltung kann eine Inspektionskamera zur Verfügung gestellt werden, die vorteilhaft einfach zu handhaben ist.

Die erfindungsgemäße Sensorvorrichtung und/oder die erfindungsgemäße Inspektionskamera sollen/soll hierbei nicht auf die oben beschriebene Anwendung und Ausführungsform beschränkt sein. Insbesondere können/kann die erfindungsgemäße Sensorvorrichtung und/oder die erfindungsgemäße Inspektionskamera zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

### Zeichnungen

Weitere Vorteile ergeben sich aus der folgenden Zeichnungsbeschreibung. In den Zeichnungen sind fünf Ausführungsbeispiele der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung einer erfindungsgemäßen Inspektionskamera,
- Fig. 2: eine schematische Darstellung einer erfindungsgemäßen Sensorvorrichtung,
- Fig. 3: eine schematische Darstellung einer weiteren Ausgestaltung einer erfindungsgemäßen Sensorvorrichtung,
- Fig. 4: eine schematische Darstellung einer Ausgestaltung einer erfindungsgemäßen Sensorvorrichtung mit einer beidseitigen Verjüngung,
- Fig. 5: eine schematische Darstellung einer alternativen Ausgestaltung einer erfindungsgemäßen Sensorvorrichtung und
- Fig. 6: eine schematische Darstellung einer Ausgestaltung einer erfindungsgemäßen Sensorvorrichtung mit einer asymmetrischen Verjüngung.

### Beschreibung der Ausführungsbeispiele

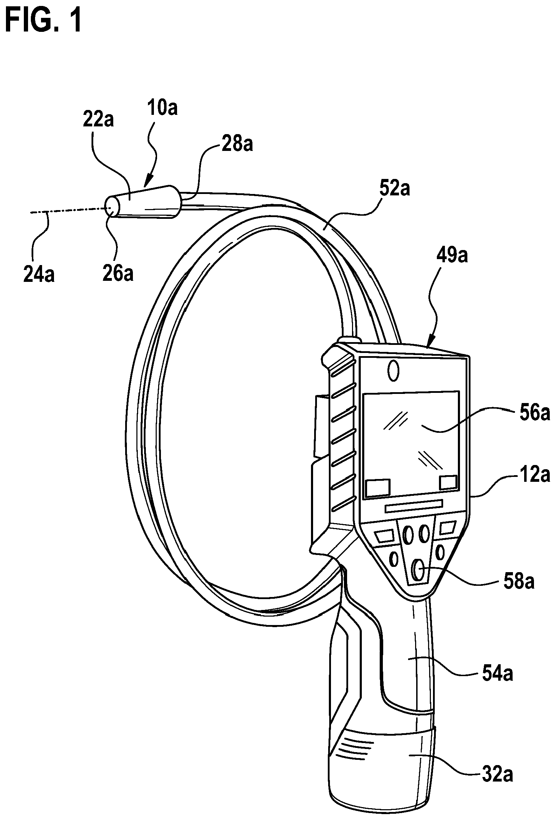

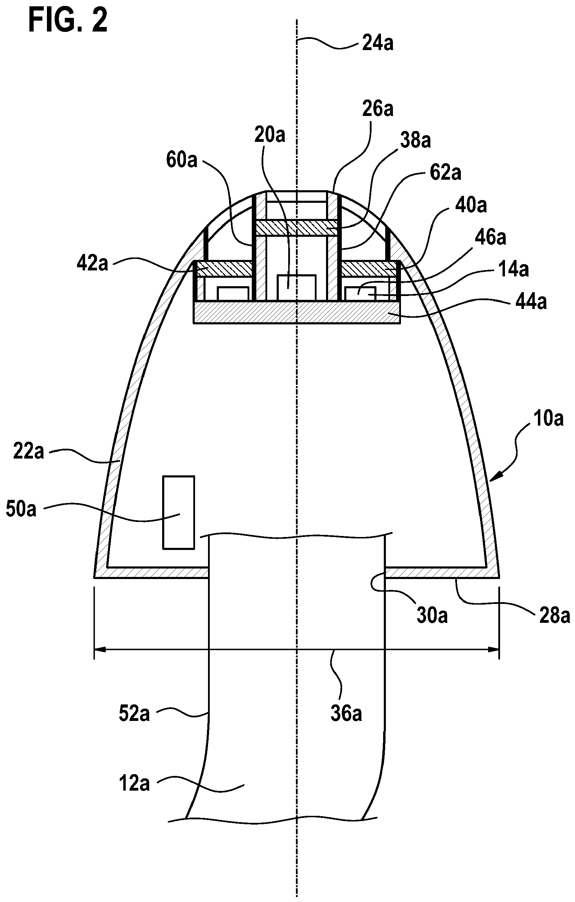

Figur 1 zeigt eine Inspektionskamera 12a, insbesondere ein Endoskop. Die Inspektionskamera 12a umfasst zumindest eine Sensorvorrichtung 10a mit einer Kameraeinheit 20a (siehe Figur 2). Die Sensorvorrichtung 10a umfasst eine Gehäuseeinheit 22a. Die Kameraeinheit 20a der Sensorvorrichtung 10a ist in der Gehäuseeinheit 22a angeordnet. Die Inspektionskamera 12a umfasst insbesondere eine Bedieneinheit 49a. Vorzugsweise umfasst die Inspektionskamera 12a zumindest eine Kabeleinheit 52a. Die Sensorvorrichtung 10a ist insbesondere an einem Ende der Kabeleinheit 52a angeordnet. Die Bedieneinheit 49a ist vorzugsweise an einem Ende der Kabeleinheit 52a angeordnet, welches insbesondere von der Sensorvorrichtung 10a abgewandt ist. Die Bedieneinheit 49a umfasst eine Energiequelle 32a. Die Energiequelle 32a ist insbesondere als Akkumulator ausgebildet. Die Bedieneinheit 49a umfasst insbesondere einen Handgriff 54a, in welchem die Energiequelle 32a angeordnet ist. Die Energiequelle 32a ist beabstandet von der Gehäuseeinheit 22a an der Sensorvorrichtung 10a angeordnet. Die Energiequelle 32a ist zu einer Energieversorgung der Sensorvorrichtung 10a, insbesondere der Kameraeinheit 20a, vorgesehen. Die Bedieneinheit 49a umfasst vorzugsweise eine Ausgabeeinheit 56a, insbesondere ein Display. Die Ausgabeeinheit 56a ist vorzugweise zu einer Ausgabe eines von der Kameraeinheit 20a erfassten Bildes vorgesehen. Die Bedieneinheit 49a umfasst zumindest ein Bedienelement 58a zu einem Bedienen der Sensorvorrichtung 10a und/oder der Bedieneinheit 49a, beispielsweise zu einem Abspeichern eines von der Kameraeinheit 20a erfassten Einzelbildes, zu einem An- und/oder Ausschalten der Kameraeinheit 20a oder dergleichen.

Figur 2 zeigt die Sensorvorrichtung 10a. Die Sensorvorrichtung 10a umfasst zumindest eine Beleuchtungseinheit 14a. Die Beleuchtungseinheit 14a ist zu einer Beleuchtung eines Untersuchungsobjekts vorgesehen. Die Sensorvorrichtung 10a umfasst die Kameraeinheit 20a. Die Kameraeinheit 20a ist zu einer Erfassung des Untersuchungsobjekts vorgesehen. Die Sensorvorrichtung 10a umfasst die Gehäuseeinheit 22a. Die Kameraeinheit 20a und die Beleuchtungseinheit 14a sind in der Gehäuseeinheit 22a angeordnet. Die Gehäuseeinheit 22a ist entlang einer Längsachse 24a der Gehäuseeinheit 22a verjüngend ausgebildet.

Die Gehäuseeinheit 22a weist bezüglich der Längsachse 24a einen vorderen Gehäuseabschnitt 26a und einen hinteren Gehäuseabschnitt 28a auf. Die Gehäuseeinheit 22a weist eine an dem hinteren Gehäuseabschnitt 28a angeordnete Kabeldurchführung 30a auf. Die Kabeldurchführung 30a ist zu einem Anschluss der Kameraeinheit 20a und/oder der Beleuchtungseinheit 14a an die Energiequelle 32a der Inspektionskamera 12a vorgesehen. Insbesondere ist die Kabeldurchführung 30a zu einer Aufnahme der Kabeleinheit 52a, insbesondere zumindest einer Datenleitung und/oder zumindest einer Stromleitung der Kabeleinheit 52a, vorgesehen. Der vordere Gehäuseabschnitt 26a ist entlang der Längsachse 24a in einer von dem hinteren Gehäuseabschnitt 28a abgewandten Richtung verjüngend ausgebildet. Insbesondere ist die Gehäuseeinheit 22a über eine gesamte Länge der Gehäuseeinheit 22a parallel zu der Längsachse 24a verjüngend ausgebildet. Beispielhaft ist die Gehäuseeinheit 22a rotationsparaboloidförmig ausgebildet. Insbesondere ist ein Scheitelpunkt einer eine Außenwand der Gehäuseeinheit 22a beschreibenden Parabel in dem vorderen Gehäuseabschnitt 26a angeordnet. Eine maximale Quererstreckung 36a der Gehäuseeinheit 22a senkrecht zur Längsachse 24a ist insbesondere in einer Ebene mit der Kabeldurchführung 30a angeordnet.

Die Sensorvorrichtung 10a umfasst insbesondere eine in der Gehäuseeinheit 22a angeordnete Montageplatte 44a, insbesondere eine Leiterplatte, an welcher zumindest die Kameraeinheit 20a montiert ist. Die Beleuchtungseinheit 14a umfasst vorzugsweise zumindest ein Beleuchtungselement 46a, insbesondere eine LED. Vorzugsweise umfasst die Beleuchtungseinheit 14a mehrere Beleuchtungselemente 46a, welche kreisförmig insbesondere in regelmäßigen Abständen um eine Blickrichtung der Kameraeinheit 20a herum angeordnet sind. Das zumindest eine Beleuchtungselement 46a ist insbesondere auf der gleichen Seite der Montageplatte 44a wie die Kameraeinheit 20a an der Montageplatte 44a angeordnet. Eine Blickrichtung der Kameraeinheit 20a ist insbesondere parallel, insbesondere koaxial, zu der Längsachse 24a ausgerichtet. Die Sensorvorrichtung 10a umfasst zumindest ein die Gehäuseeinheit 22a verschließendes Schutzfenster 38a zu einem Schutz der Kameraeinheit 20a. Die Sensorvorrichtung 10a umfasst zumindest ein weiteres die Gehäuseeinheit 22a verschließendes Schutzfenster 40a, 42a zu einem Schutz der Beleuchtungseinheit 14a. Das Schutzfenster 38a und das weitere Schutzfenster 40a, 42a sind separat voneinander ausgebildet. Das Schutzfenster 38a und das weitere Schutzfenster 40a, 42a sind in einer Richtung parallel zu der Längsachse 24a versetzt zueinander angeordnet. Insbesondere bildet die Gehäuseeinheit 22a zumindest ein Blockierelement 60a, 62a aus, welches an einer Seitenwand des Schutzfensters 38a und/oder des weiteren Schutzfensters 40a, 42a angeordnet ist. Bevorzugt erstreckt sich das Blockierelement 60a, 62a insbesondere von einer Außenwand der Gehäuseeinheit 22a bis zur der Montageplatte 44a.

Die Sensorvorrichtung 10a umfasst einen Ausrichtungssensor 50a zu einer Bestimmung einer räumlichen Ausrichtung der Kameraeinheit 20a. Der Ausrichtungssensor 50a ist insbesondere innerhalb der Gehäuseeinheit 22a angeordnet.

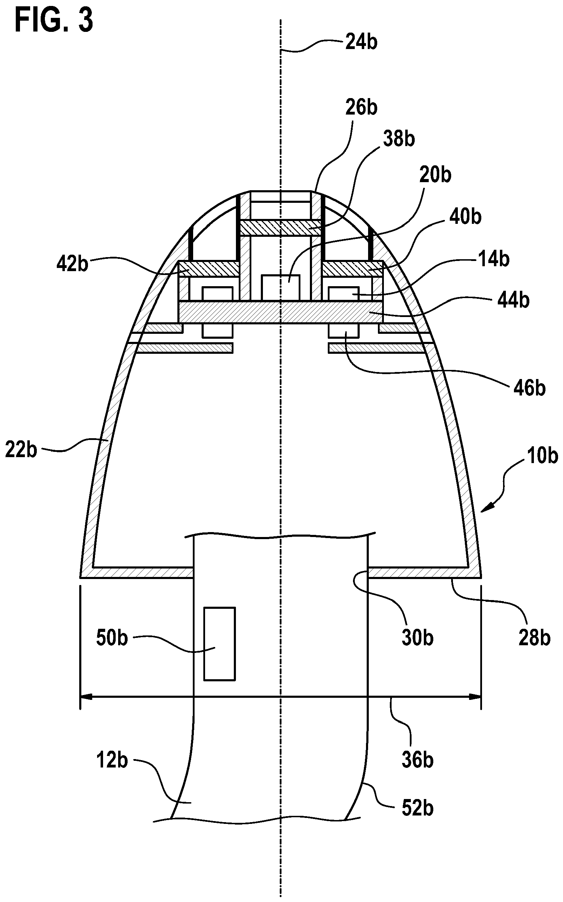

In den Figuren 3 bis 6 sind vier weitere Ausführungsbeispiele der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Figuren 1 bis 2, verwiesen werden kann. Zur Unterscheidung der Ausführungsbeispiele ist der Buchstabe a den Bezugszeichen des Ausführungsbeispiels in den Figuren 1 bis 2 nachgestellt. In den Ausführungsbeispielen der Figuren 3 bis 6 ist der Buchstabe a durch die Buchstaben b bis e ersetzt.

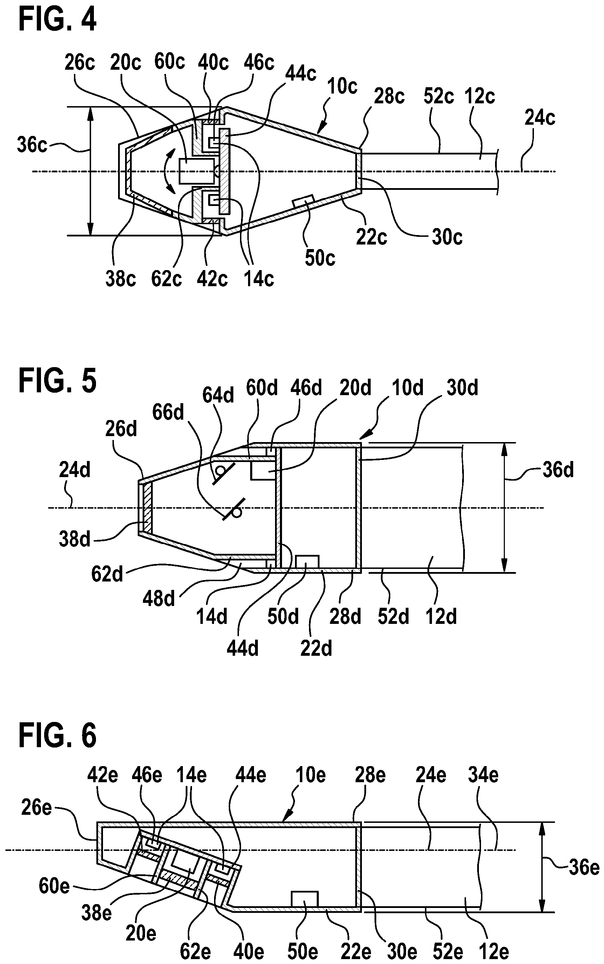

Figur 4 zeigt eine Sensorvorrichtung 10b für eine Inspektionskamera 12b. Die Sensorvorrichtung 10b umfasst zumindest eine Beleuchtungseinheit 14b. Die Beleuchtungseinheit 14b ist zu einer Beleuchtung eines Untersuchungsobjekts vorgesehen. Die Sensorvorrichtung 10b umfasst eine Kameraeinheit 20b. Die Kameraeinheit 20b ist zu einer Erfassung des Untersuchungsobjekts vorgesehen. Die Sensorvorrichtung 10b umfasst eine Gehäuseeinheit 22b. Die Kameraeinheit 20b und die Beleuchtungseinheit 14b sind in der Gehäuseeinheit 22b angeordnet. Die Gehäuseeinheit 22b ist entlang einer Längsachse 24b der Gehäuseeinheit 22b verjüngend ausgebildet. Die Sensorvorrichtung 10b umfasst eine in der Gehäuseeinheit 22b angeordnete Montageplatte 44b, insbesondere eine Leiterplatte. An der Montageplatte 44b ist zumindest die Kameraeinheit 20b montiert. Die Beleuchtungseinheit 14b umfasst zumindest ein Beleuchtungselement 46b, welches auf einer von der Kameraeinheit 20b abgewandten Seite der Montageplatte 44b angeordnet ist.

Die Sensorvorrichtung 10b umfasst einen Ausrichtungssensor 50b zu einer Bestimmung einer räumlichen Ausrichtung der Kameraeinheit 20b. Der Ausrichtungssensor 50b ist insbesondere in eine Kabeleinheit 52b der Inspektionskamera 12b integriert. Vorzugsweise ist der Ausrichtungssensor 50b weniger weit von der Gehäuseeinheit 22b entfernt an oder in der Kabeleinheit 52b angeordnet, als eine maximale Längserstreckung der Gehäuseeinheit 22b parallel zu der Längsachse 24b.

Bezüglich weiterer Merkmale der Sensorvorrichtung 10b sei insbesondere auf die Figuren 1 bis 2 und deren Beschreibung verwiesen.

Figur 4 zeigt eine Sensorvorrichtung 10c für eine Inspektionskamera 12c. Die Sensorvorrichtung 10c umfasst zumindest eine Beleuchtungseinheit 14c. Die Beleuchtungseinheit 14c ist zu einer Beleuchtung eines Untersuchungsobjekts vorgesehen. Die Sensorvorrichtung 10c umfasst eine Kameraeinheit 20c. Die Kameraeinheit 20c ist zu einer Erfassung des Untersuchungsobjekts vorgesehen. Die Sensorvorrichtung 10c umfasst eine Gehäuseeinheit 22c. Die Kameraeinheit 20c und die Beleuchtungseinheit 14c sind in der Gehäuseeinheit 22c angeordnet. Die Gehäuseeinheit 22c ist entlang einer Längsachse 24c der Gehäuseeinheit 22c verjüngend ausgebildet. Die Gehäuseeinheit 22c weist bezüglich der Längsachse 24c einen vorderen Gehäuseabschnitt 26c und einen hinteren Gehäuseabschnitt 28c auf. Die Gehäuseeinheit 22c weist eine an dem hinteren Gehäuseabschnitt 28c angeordnete Kabeldurchführung 30c zu einem Anschluss der Kameraeinheit 20c und/oder der Beleuchtungseinheit 14c an eine Energiequelle der Inspektionskamera 12c auf. Der hintere Gehäuseabschnitt 28c ist entlang der Längsachse 24c in einer von dem vorderen Gehäuseabschnitt 26c abgewandten Richtung verjüngend ausgebildet. Der vordere Gehäuseabschnitt 26c ist entlang der Längsachse 24c in einer von dem hinteren Gehäuseabschnitt 28c abgewandten Richtung verjüngend ausgebildet. Insbesondere weist die Gehäuseeinheit 22c in einer zur Längsachse 24c parallelen Ebene einen rautenförmigen Querschnitt auf.

Eine Blickrichtung der Kameraeinheit 20c ist unter einem, insbesondere einstellbaren, spitzen Winkel zu der Längsachse 24c ausgerichtet, um das Untersuchungsobjekt durch eine die Gehäuseeinheit 22c verjüngende Schrägfläche der Gehäuseeinheit 22c hindurch zu erfassen. Insbesondere umfasst die Kameraeinheit 20c zumindest ein schwenkbar gelagertes Kameraelement und ein Antriebselement zu einer Bewegung des Kameraelements relativ zu der Längsachse 24c.

Bezüglich weiterer Merkmale der Sensorvorrichtung 10c sei insbesondere auf die Figuren 1 bis 3 und deren Beschreibung verwiesen.

Figur 5 zeigt eine Sensorvorrichtung 10d für eine Inspektionskamera 12d. Die Sensorvorrichtung 10d umfasst zumindest eine Beleuchtungseinheit 14d. Die Beleuchtungseinheit 14d ist zu einer Beleuchtung eines Untersuchungsobjekts vorgesehen. Die Sensorvorrichtung 10d umfasst eine Kameraeinheit 20d. Die Kameraeinheit 20d ist zu einer Erfassung des Untersuchungsobjekts vorgesehen. Die Sensorvorrichtung 10d umfasst eine Gehäuseeinheit 22d. Die Kameraeinheit 20d und die Beleuchtungseinheit 14d sind in der Gehäuseeinheit 22d angeordnet. Die Gehäuseeinheit 22d ist entlang einer Längsachse 24d der Gehäuseeinheit 22d verjüngend ausgebildet. Die Gehäuseeinheit 22d bildet zumindest einen Lichtleiter 48d aus, welcher von der Beleuchtungseinheit 14d parallel oder quer zur Längsachse 24d wegführt.

Eine Blickrichtung der Kameraeinheit 20d ist unter einem, insbesondere einstellbaren, spitzen Winkel zu der Längsachse 24d ausgerichtet. Insbesondere umfasst die Kameraeinheit 20d zumindest einen Mikrospiegelaktor 64d, 66d zu einer Verkippung der Blickrichtung relativ zu der Längsachse 24d.

Ein eine Kabeldurchführung 30d der Gehäuseeinheit 22d begrenzender Rand der Gehäuseeinheit 22d weist in einer zur Längsachse 24d senkrechten Ebene eine maximale Quererstreckung 36d der Gehäuseeinheit 22d senkrecht zur Längsachse 24d auf. Insbesondere schließen die Gehäuseeinheit 22d und eine Kabeleinheit 52d der Inspektionskamera 12d in einer Richtung quer zur Längsachse 24d bündig miteinander ab.

Bezüglich weiterer Merkmale der Sensorvorrichtung 10d sei insbesondere auf die Figuren 1 bis 4 und deren Beschreibung verwiesen.

Figur 6 zeigt eine Sensorvorrichtung 10e für eine Inspektionskamera 12e. Die Sensorvorrichtung 10e umfasst zumindest eine Beleuchtungseinheit 14e. Die Beleuchtungseinheit 14e ist zu einer Beleuchtung eines Untersuchungsobjekts vorgesehen. Die Sensorvorrichtung 10e umfasst eine Kameraeinheit 20e. Die Kameraeinheit 20e ist zu einer Erfassung des Untersuchungsobjekts vorgesehen. Die Sensorvorrichtung 10e umfasst eine Gehäuseeinheit 22e. Die Kameraeinheit 20e und die Beleuchtungseinheit 14e sind in der Gehäuseeinheit 22e angeordnet. Die Gehäuseeinheit 22e ist entlang einer Längsachse 24e der Gehäuseeinheit 22e verjüngend ausgebildet. Die Gehäuseeinheit 22e ist bezüglich einer zur Längsachse 24e parallelen Mittenebene 34e durch einen geometrischen Schwerpunkt der Gehäuseeinheit 22e asymmetrisch ausgebildet. Eine Blickrichtung der Kameraeinheit 20e ist unter einem festgelegten spitzen Winkel zu der Längsachse 24e ausgerichtet, um das Untersuchungsobjekt durch eine die Gehäuseeinheit 22e verjüngende Schrägfläche der Gehäuseeinheit 22e hindurch zu erfassen.

Bezüglich weiterer Merkmale der Sensorvorrichtung 10e sei insbesondere auf die Figuren 1 bis 5 und deren Beschreibung verwiesen.

## Patentansprüche

1. Sensorvorrichtung, insbesondere Sensorkopf, für eine Inspektionskamera, insbesondere Endoskop, mit zumindest einer Beleuchtungseinheit (14a; 14b; 14c; 14d; 14e) zu einer Beleuchtung eines Untersuchungsobjekts, mit zumindest einer Kameraeinheit (20a; 20b; 20c; 20d; 20e) zu einer Erfassung des Untersuchungsobjekts und mit zumindest einer Gehäuseeinheit (22a; 22b; 22c; 22d; 22e), in welcher die Kameraeinheit (20a; 20b; 20c; 20d; 20e) und die Beleuchtungseinheit (14a; 14b; 14c; 14d; 14e) angeordnet sind, **dadurch gekennzeichnet, dass** die Gehäuseeinheit (22a; 22b; 22c; 22d; 22e) entlang einer Längsachse (24a; 24b; 24c; 24d; 24e) der Gehäuseeinheit (22a; 22b; 22c; 22d; 22e) verjüngend ausgebildet ist.

2. Sensorvorrichtung nach Anspruch 1, **dadurch gekennzeichnet, dass** die Gehäuseeinheit (22a; 22b; 22c; 22d; 22e) bezüglich der Längsachse (24a; 24b; 24c; 24d; 24e) einen vorderen Gehäuseabschnitt (26a; 26b; 26c; 26d; 26e), einen hinteren Gehäuseabschnitt (28a; 28b; 28c; 28d; 28e) und eine an dem hinteren Gehäuseabschnitt (28a; 28b; 28c; 28d; 28e) angeordnete Kabeldurchführung (30a) zu einem Anschluss der Kameraeinheit (20a; 20b; 20c; 20d; 20e) und/oder der Beleuchtungseinheit (14a; 14b; 14c; 14d; 14e) an eine Energiequelle (32a) der Inspektionskamera umfasst, wobei der vordere Gehäuseabschnitt (26a; 26b; 26c; 26d; 26e) entlang der Längsachse (24a; 24b; 24c; 24d; 24e) in einer von dem hinteren Gehäuseabschnitt (28a; 28b; 28c; 28d; 28e) abgewandten Richtung verjüngend ausgebildet ist.

3. Sensorvorrichtung nach Anspruch 1 oder 2, **dadurch gekennzeichnet, dass** die Gehäuseeinheit (22e) bezüglich einer zur Längsachse (24e) parallelen Mittenebene (34e) durch einen geometrischen Schwerpunkt der Gehäuseeinheit (22e) asymmetrisch ausgebildet ist.

4. Sensorvorrichtung nach einem der vorhergehenden Ansprüche, **dadurch gekennzeichnet, dass** ein eine Kabeldurchführung (30d; 30e) der Gehäuseeinheit (22d; 22e) begrenzender Rand der Gehäuseeinheit (22d; 22e) in einer zur Längsachse (24d; 24e) senkrechten Ebene eine maximale Quererstreckung (36d; 36e) der Gehäuseeinheit (22d; 22e) senkrecht zur Längsachse (24d; 24e) aufweist.

5. Sensorvorrichtung nach einem Ansprüche 1 bis 3, **dadurch gekennzeichnet, dass** die Gehäuseeinheit (22c) bezüglich der Längsachse (24c) einen vorderen Gehäuseabschnitt (26c), einen hinteren Gehäuseabschnitt (28c) und eine an dem hinteren Gehäuseabschnitt (28c) angeordnete Kabeldurchführung (30c) zu einem Anschluss der Kameraeinheit (20c) und/oder der Beleuchtungseinheit (14c) an eine Energiequelle der Inspektionskamera umfasst, wobei der hintere Gehäuseabschnitt (28c) entlang der Längsachse (24c) in einer von dem vorderen Gehäuseabschnitt (26c) abgewandten Richtung verjüngend ausgebildet ist.

6. Sensorvorrichtung nach einem der vorhergehenden Ansprüche, **gekennzeichnet durch** zumindest ein die Gehäuseeinheit (22a; 22b; 22c; 22e) verschließendes Schutzfenster (38a; 38b; 38c; 38e) zu einem Schutz der Kameraeinheit (20a; 20b; 20c; 20e) und mit zumindest einem weiteren Schutzfenster (40a, 42a; 40b, 42b; 40c, 42c; 40e, 42e) zu einem Schutz der Beleuchtungseinheit (14a; 14b; 14c; 14e), wobei das Schutzfenster (38a; 38b; 38c; 38e) und das weitere Schutzfenster (40a, 42a; 40b, 42b; 40c, 42c; 40e, 42e) separat voneinander ausgebildet sind.

7. Sensorvorrichtung nach Anspruch 5, **dadurch gekennzeichnet, dass** das Schutzfenster (38a; 38b; 38c) und das weitere Schutzfenster (40a, 42a; 40b, 42b; 40c, 42c) in einer Richtung parallel zu der Längsachse (24a; 24b; 24c) versetzt zueinander angeordnet sind.

8. Sensorvorrichtung nach einem der vorhergehenden Ansprüche, **dadurch gekennzeichnet, dass** eine Blickrichtung der Kameraeinheit (20c; 20d; 20e) unter einem, insbesondere einstellbaren, spitzen Winkel zu der Längsachse (24c; 24d; 24e) ausgerichtet ist, um das Untersuchungsobjekt durch eine die Gehäuseeinheit (22c; 22d; 22e) verjüngende Schrägfläche der Gehäuseeinheit (22c; 22d; 22e) hindurch zu erfassen.

9. Sensorvorrichtung nach einem der vorhergehenden Ansprüche, **gekennzeichnet durch** eine in der Gehäuseeinheit (22b) angeordnete Montageplatte (44b), insbesondere Leiterplatte, an welcher zumindest die Kameraeinheit (20b) montiert ist, wobei die Beleuchtungseinheit (14b) zumindest ein Beleuchtungselement (46b) umfasst, welches auf einer von der Kameraeinheit (20b) abgewandten Seite der Montageplatte (44b) angeordnet ist.

10. Sensorvorrichtung nach einem der vorhergehenden Ansprüche, **dadurch gekennzeichnet, dass** die Gehäuseeinheit (22d) zumindest einen Lichtleiter (48d) ausbildet, welcher von der Beleuchtungseinheit (14d) parallel oder quer zur Längsachse (24d) wegführt.

11. Sensorvorrichtung nach einem der vorhergehenden Ansprüche, **gekennzeichnet durch** zumindest einen Ausrichtungssensor (50a; 50b; 50c; 50d; 50e) zu einer Bestimmung einer räumlichen Ausrichtung der Kameraeinheit (20a; 20b; 20c; 20d; 20e).

12. Inspektionskamera, insbesondere Endoskop, mit zumindest einer Sensorvorrichtung nach einem der vorhergehenden Ansprüche und mit zumindest einer von der Gehäuseeinheit (22a; 22b; 22c; 22d; 22e) beabstandet angeordneten Energiequelle (32a) zu einer Energieversorgung der Kameraeinheit (20a; 20b; 20c; 20d; 20e) und/oder der Beleuchtungseinheit (14a; 14b; 14c; 14d; 14e) mit elektrischer Energie.
